# EUROPÄISCHE PATENTSCHRIFT

(11) **EP 1 574 188 B1**
(45) Veröffentlichungstag und Bekanntmachung des Hinweises auf die Patenterteilung: **10.11.2010**
(21) Anmeldenummer: 05005019.4
(22) Anmeldetag: 08.03.2005
(51) Int. Cl.: A61F 5/00

(54) **Implantat zur Behandlung von Fettsucht**
Implant for treatment of obesity
Implant pour le traitement de l'obésité

(30) Priorität: 09.03.2004 DE 102004011764
(43) Veröffentlichungstag der Anmeldung: 14.09.2005
(73) Patentinhaber: NOVINEON HEALTHCARE TECHNOLOGY PARTNERS GMBH, 72074 Tübingen (DE)
(72) Erfinder: Kalanovic, Daniel, Dr. med., 70180 Stuttgart (DE); Ho, Chi-Nghia, 72074 Tübingen (DE); Schostek, Sebastian, 72074 Tübingen (DE); Schurr, Marc Oliver, Prof. Dr. med., 72074 Tübingen (DE)
(74) Vertreter: Winter, Brandl, Fürniss, Hübner, Röss, Kaiser, Polte - Partnerschaft

(56) Entgegenhaltungen:
- WO-A-02/096325
- US-A- 5 306 300
- US-A- 5 861 036

## Beschreibung

Die vorliegende Erfindung bezieht sich auf eine Vorrichtung zur Behandlung krankhafter Fettsucht und insbesondere auf ein medizinisches Implantat, das dazu angepasst ist, die natürliche Speisepassage im Verdauungstrakt zu verändern und wahlweise zu umgehen. Im Gegensatz zu rein chirurgischen Verfahren der Nahrungsumleitung, erlauben die Eigenschaften des vorliegenden Implantats eine einstellbare Umleitung von Nahrung in den Dünndarm bzw. in den Magen (natürlicher Verdauungsweg). Da das Verhältnis dieser Umleitung im Behandlungsverlauf durch den Arzt z.B. mithilfe eines Endoskops einstellbar ist, kann durch Verwendung des Implantats eine Balance zwischen therapeutischem Effekt (Gewichtsverlust) und Nebenwirkungen (übermäßige Malabsorption) erstmals individuell auf den jeweiligen Patienten angepasst werden.
Massives Übergewicht wird heutzutage durch den so genannten Body Mass Index (BMI) definiert. Dieser ergibt sich aus dem Körpergewicht in Kilogramm (kg) dividiert durch das Quadrat der Körpergröße in Metern (m). Ein BMI von mehr als 40 entspricht massivem Übergewicht. Massives Übergewicht führt bei Menschen zu einer Anzahl gesundheitlicher Folgen wie zum Beispiel Herzkreislauferkrankungen, Diabetes und Schäden am Bewegungsapparat. Bei extremen Formen der Fettsucht wird trotz aller Bemühungen in der Regel langfristig nur eine Reduktion um einige Kilogramm erreicht, was kaum ins Gewicht fällt. In diesen extremen Fällen kann sich die Indikation zur chirurgischen Therapie ergeben. Bei der chirurgischen Therapie von massivem Übergewicht werden in heutiger Zeit Operationen durchgeführt, von denen sich das "Gastric-Banding" und der "Gastric-Bypass" durchgesetzt haben.

### Gastric-Banding

Bei diesem Eingriff wird der Eingangsbereich des Magens durch ein implantiertes Kunststoffband abgeschnürt, wodurch eine Magentasche entsteht, die nur über einen kleinen Auslass mit dem übrigen Magenbereich in Verbindung ist.

Dieser Eingriff kann jedoch dazu führen, dass der Patient nach der Operation vermehrt hochkalorisch isst oder dass die abgeschnürte Magentasche ausbaucht und expandiert, so dass ein gewisser Wiederanstieg des Gewichts wahrscheinlich ist. Des Weiteren besteht die Möglichkeit, dass das Silikonband verrutscht oder in den Magen durchbricht.

### Gastric-Bypass

Bei diesem Eingriff wird im Eingangsbereich des Magens mithilfe von Klammernahtvorrichtungen eine Magentasche chirurgisch abgesetzt. Dabei wird der Magen des Patienten vollständig durchtrennt und die gebildete Magentasche ist nicht mehr mit dem übrigen Magenbereich, sondern mit der anastomosierten Dünndarmschlinge in Verbindung, die an der Magentasdhe hochgezogen und fixiert ist. Die Nahrung passiert die Speiseröhre, die Magentasche und fließt dann in die Dünndarmschlinge ab, wobei der Restmagen und der Zwölffingerdarm von der Nahrungspassage, aber auch von endoskopischen Untersuchungen ausgeschlossen sind. Der Gastric-Bypass führt zu einer größeren Gewichtsabnahme als das Gastric-Banding.

Der Eingriff gilt jedoch als irreversibel, was im Falle von späteren Komplikationen wie zum Beispiel schwer kontrollierbaren Malabsorptionsfolgen nachteilig ist. Es besteht ferner ein Risiko, dass Nähte an den Klammernahtvorrichtungen undicht werden, wodurch eine weitere Operation erforderlich wäre. Auch eine gastroskopische Untersuchung des abgesetzten Magens und Zwölffingerdarms ist nach dem Eingriff nicht mehr möglich.

Aus der WO 02/09 63 25 A ist ein medizinisches Implantat für den Gastric-Bypass bekannt, bestehend aus einem mittig angeordneten Hohlkörper als Magenattrappe, in den ein Eingangsschlauch und ein Ausgangsschlauch an sich gegenüberliegenden Stellen des Hohlkörpers einmünden. Der Eingangsschlauch ist in der Speiseröhre und des Ausgangsschlauch im Dünndarm des Patienten dichtend fixierbar. Hierdurch wird der Patientenmagen vollständig umgangen und aufgenommene Speisen von der Speiseröhre direkt und vollständig in den Dünndarm eingeleitet.

Die US-A-5 861 036 offenbart einen konisch geformten Eingangsschlauch, der in der Speiseröhre fixierbar ist und in den Magen eines Patienten führt.

Schließlich zeigt die US-A-5 306 300 einen in den Dünndarm eines Patienten einführbaren Membranschlauch mit einer Anzahl von seitlichen Öffnungen zur kontrollierten Abgabe von Speisebrei in den Dünndarm.

Es ist Aufgabe der Erfindung, eine Vorrichtung zur Behandlung krankhafter Fettsucht vorzusehen, die die Fettsucht nachhaltig bekämpft, dabei schonender als gegenwärtige Verfahren ist und einen reversiblen Eingriff an den Patienten ermöglicht.

Bisherige hohlförmige Implantate zur Behandlung krankhafter Fettsucht (Patente Nr. WO 88 05671 A, US 4,315,509 A, US 5,306,300) beschreiben eine Struktur, die die durch die Speiseröhre kommende Nahrung aufnehmen und den natürlichen gastrointestinalen Transportweg weiterführen sollen. Innerhalb dieser Implantate soll die Nahrung von den Verdauungsmechanismen getrennt und die Nährstoffaufnahme dadurch beeinträchtigt werden.

Im Gegensatz zu den oben genannten Implantatkonzepten wird die Aufgabe durch die beschriebene Vorrichtung gemäß Anspruch 1 einerseits durch eine Umleitung von aufgenommener Nahrung durch eine vorgesehene Öffnung in den Dünndarm erreicht. Andererseits erlaubt das Implantat eine graduell einstellbare Nahrungspassage in den vollständig erhaltenen Magen und dadurch einen skalierbaren natürlichen gastrointestinalen Transportweg. Vorteilhafte Weiterbildungen sind in den abhängigen Ansprüchen dargelegt.

Die Vorrichtung ist ein medizinisches Implantat mit einem in den Magen des Patienten implantierbaren Hohlkörper zur temporären Aufnahme von Nahrung mit einem ersten röhrenartigen Endabschnitt, einem zweiten röhrenartigen Endabschnitt sowie mit mindestens einer dritten seitlichen Öffnung zwischen dem ersten und dem zweiten röhrenartigen Endabschnitt. Der erste röhrenartige Endabschnitt ist so bemessen, dass er in die Speiseröhre des Patienten dichtend einpassbar ist. Der zweite röhrenartige Endabschnitt ist so bemessen, dass er mit der Dünndarmschlinge des Patienten dichtend verbindbar ist. Die dritte seitliche Öffnung weist einen schlauchartigen Fortsatz auf und stellt eine Kommunikation zwischen dem Hohlkörper und dem Mageninnenraum her, so dass ein Teil der Nahrung vom Hohlkörper in den Magen passieren kann. Des Weiteren wird die endoskopische Inspektion des Magens durch eine seitliche Öffnung ermöglicht.

Gemäß einer vorteilhaften Weiterbildung hat das Implantat Fixiervorrichtungen an dem ersten und dem zweiten röhrenartigen Endabschnitt zum Fixieren der Endabschnitte an Organteile des Patienten.

Gemäß einer weiteren vorteilhaften Weiterbildung hat das Implantat ein hohles Mittelteil zwischen dem ersten und dem zweiten Endabschnitt, das relativ zu den Endabschnitten ausgebaucht ist und mit diesen in Verbindung steht. Dieser mittlere Abschnitt des Implantats nimmt die durch den ersten röhrenartigen Endabschnitt kommenden Substanzen auf und leitet diese selektiv weiter entweder durch den zweiten röhrenförmigen Endabschnitt zu der Dünndarmschlinge oder durch eine seitliche Öffnung in den Mageninnenraum.

Gemäß einer weiteren vorteilhaften Weiterbildung besteht das hohle Mittelteil des Implantats aus einem flexiblen Material oder einer flexiblen Struktur, um einerseits die über den ersten röhrenförmigen Endabschnitt kommenden Substanzen problemlos aufzunehmen und andererseits die mechanische Einordnung in die menschliche Anatomie zu gewährleisten.

Gemäß einer weiteren vorteilhaften Weiterbildung ist mindestens eine seitliche Öffnung im Bereich des hohlen Mittelteils lokalisiert. Aus dem hohlen Mittelteil können Substanzen oder medizinische Instrumente über diese seitliche Öffnung direkt in den Mageninnenraum gelangen.

Gemäß einer weiteren vorteilhaften Weiterbildung besteht der schlauchartige Fortsatz der seitlichen Öffnung aus einem leicht verformbaren Material oder einer leicht verformbaren Struktur, sodass sich der schlauchartige Fortsatz zum Zwecke des Verschließens leicht nach innen stülpen und mit einer mechanischen Verschlussvorrichtung ausstatten lässt.

Gemäß einer weiteren vorteilhaften Weiterbildung weist der schlauchförmige Fortsatz der seitlichen Öffnung eine Struktur auf, die das Greifen des schlauchförmigen Fortsatzes mit medizinischen Instrumenten erleichtert. Bei einem endoskopischen Eingriff kann der schlauchartige Fortsatz einer seitlichen Öffnung so leichter mit mechanischen Instrumenten wie z.B. einer Zange gegriffen und somit zum Zwecke des Verschlusses der seitlichen Öffnung in den Innenraum des Hohlkörpers gezogen werden.

Gemäß einer weiteren vorteilhaften Weiterbildung ist der schlauchförmige Fortsatz einer seitlichen Öffnung zumindest teilweise verschließbar. Dies kann durch Anbringen einer mechanischen Vorrichtung an den in den Hohlraum eingestülpten schlauchartigen Fortsatz geschehen. Dadurch kann das Verhältnis der Passage von Substanzen in den Magen und in die Dünndarmschlinge justiert werden.

Die Erfindung wird zusammen mit ihren Vorteilen aus der nachfolgenden Beschreibung des Ausführungsbeispiels zusammen mit den beigefügten Zeichnungen ersichtlich.

Fig. 1 zeigt ein Ausführungsbeispiel der vorliegenden Erfindung; und

Fig. 2 zeigt das Ausführungsbeispiel der vorliegenden Erfindung, während es im Magen eines Patienten implantiert ist.

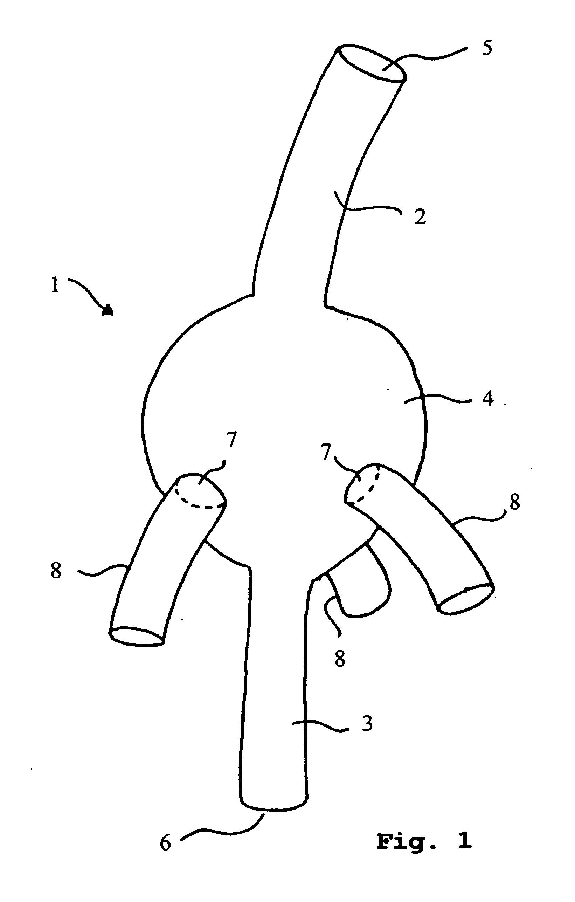

In der Fig. 1 ist ein medizinisches Implantat zur Behandlung von krankhafter Fettsucht gemäß einem Ausführungsbeispiel der vorliegenden Erfindung gezeigt. Dieses Implantat ist ein flexibler Hohlkörper 1. Der Hohlkörper 1 hat einen ersten röhrenartigen Endabschnitt 2 und einen zweiten röhrenartigen Endabschnitt 3. Der erste röhrenartige Endabschnitt 2 ist so bemessen, dass er in die Speiseröhre des Patienten einpassbar ist. Der zweite röhrenartige Endabschnitt 3 ist so bemessen, dass er in die Dünndarmschlinge des Patienten einpassbar ist.

Der erste und der zweite röhrenartige Endabschnitt 2, 3 haben jeweils eine erste Öffnung 5 bzw. zweite Öffnung 6.

An dem ersten und dem zweiten röhrenartigen Endabschnitt 2, 3 befinden sich (nicht gezeigte) Fixiervorrichtungen, durch die die Endabschnitte 2, 3 an Organteile des Patienten fixierbar sind.

Der Hohlkörper 1 hat ein hohles flexibles Mittelteil 4, das relativ zu dem ersten und dem zweiten Endabschnitt 2, 3 ausgebaucht ist und mit dem ersten und dem zweiten Endabschnitt 2, 3 in Verbindung ist. Dieses Mittelteil 4 definiert das spätere künstliche Magenvolumen des Patienten. Da das Mittelteil 4 flexibel ist, kann sich das künstliche Magenvolumen je nach Füllungsgrad des Mittelteils 4 in gewissem Maße ausdehnen. Das Mittelteil 4 weist drei seitliche Öffnungen 7 auf, die in regelmäßigen Abständen auf gleicher Höhe ringförmig am Mittelteil 4 lokalisiert sind. Diese seitlichen Öffnungen 7 weisen jeweils einen schlauchförmigen Fortsatz 8 auf.

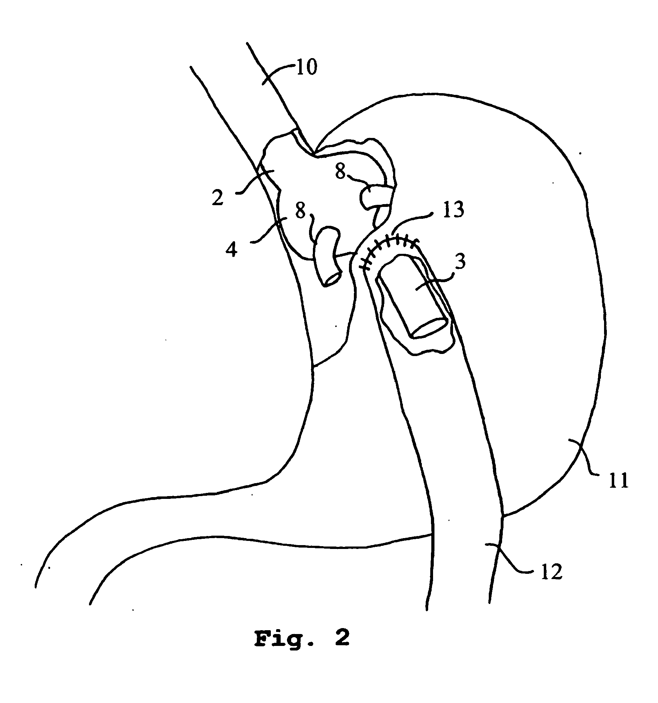

Fig. 2 zeigt, wie das medizinische Implantat zur Behandlung von krankhafter Fettsucht gemäß dem Ausführungsbeispiel der vorliegenden Erfindung im Magen implantiert ist.

Hier ist die Anordnung des Implantats im Magen klar erkennbar. Der erste röhrenartige Endabschnitt 2 ist in der Speiseröhre 10 eingepasst, während der zweite röhrenartige Endabschnitt 3 in eine abführende Dünndarmschlinge 12 eingepasst ist. Die Dünndarmschlinge 12 wurde im Voraus an die Magenwand durch z.B. Klammernahtvorrichtungen 13 zirkulär geklammert, nachdem an dieser Stelle ein passender Durchbruch in der Magenwand vorgesehen wurde. Auf diese Weise ist durch das Implantat zwischen der Speiseröhre 10 und dem Dünndarm 12 eine Umgehung verwirklicht, die den Magen 11 und den Zwölffingerdarm (nicht gezeigt) vollständig umgehen kann.

Die schlauchartigen Fortsätze 8 der seitlichen Öffnungen 7 zeigen in unterschiedliche Richtungen. Dabei ist es vorgesehen, dass einzelne schlauchartige Fortsätze 8 die Nahrungspassage in den Magen 11 unterschiedlich gut ermöglichen. Damit ergibt sich eine individuell einstellbare Nahrungsverteilung zwischen Magen 11 und Dünndarmschlinge 12.

Die Nahrung gelangt aus der Speiseröhre 10 durch die erste Öffnung 5 und den ersten röhrenartigen Endabschnitt 2 in das Mittelteil 4. Vom Mittelteil 4 aus kann die Nahrung entweder durch den zweiten röhrenartigen Endabschnitt 3 und durch die zweite Öffnung 6 in die Dünndarmschlinge 12 gelangen, oder durch eine seitliche Öffnung 7 und einen schlauchförmigen Fortsatz 8 in den Magen 11. Das Volumenverhältnis zwischen der in die Dünndarmschlinge 12 und der in den Magen 11 weitergeleiteten Nahrung bestimmt den therapeutischen Effekt des Implantats. Dieses Verhältnis ist durch Verschließen eines oder mehrerer seitlicher Öffnungen 7 justierbar.

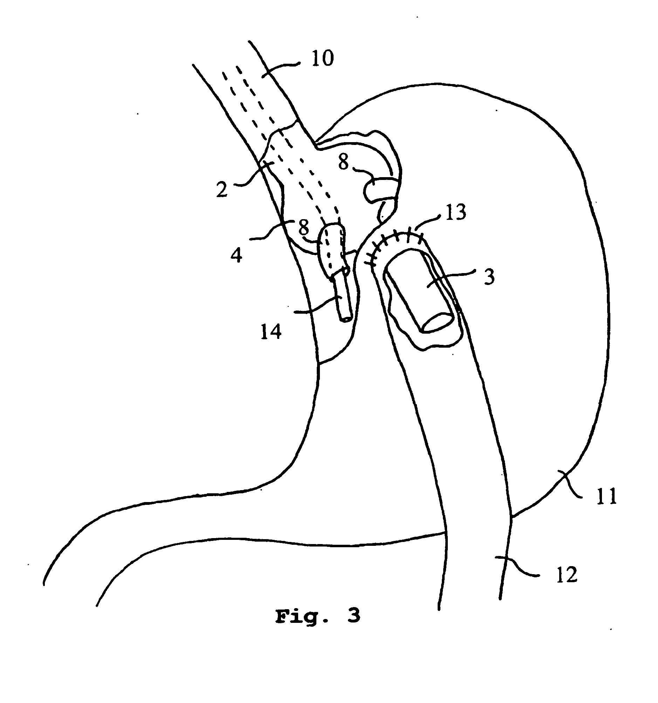

Fig. 3 zeigt die gastroskopische Inspektion des Magens 11 mithilfe eines Endoskops 14.

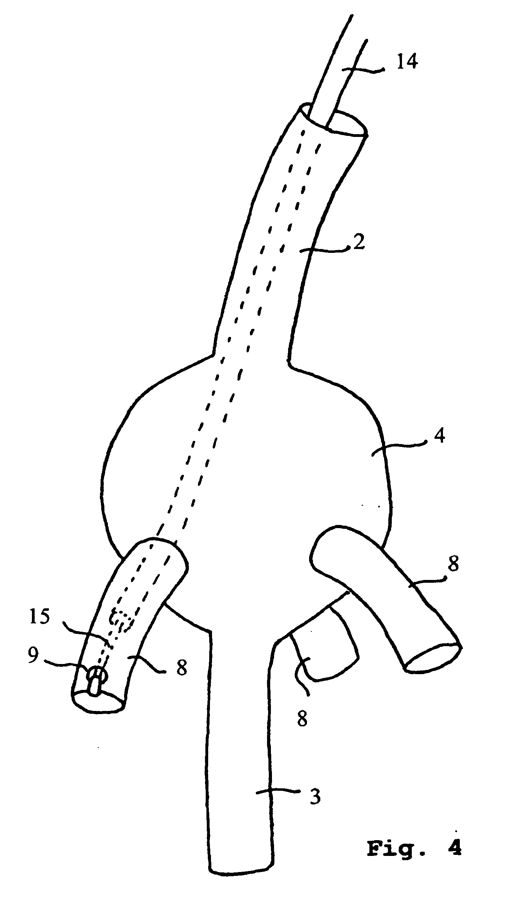

Fig. 4 zeigt das Greifen eines schlauchartigen Fortsatzes 8 einer seitlichen Öffnung 7. Die an der Spitze des schlauchförmigen Fortsatzes 8 angebrachte Struktur 9 ermöglicht ein einfaches Greifen des schlauchförmigen Fortsatzes 8 durch eine endoskopische Greifzange 15. Diese Prozedur kann unter Verwendung eines Endoskops 14 durchgeführt werden.

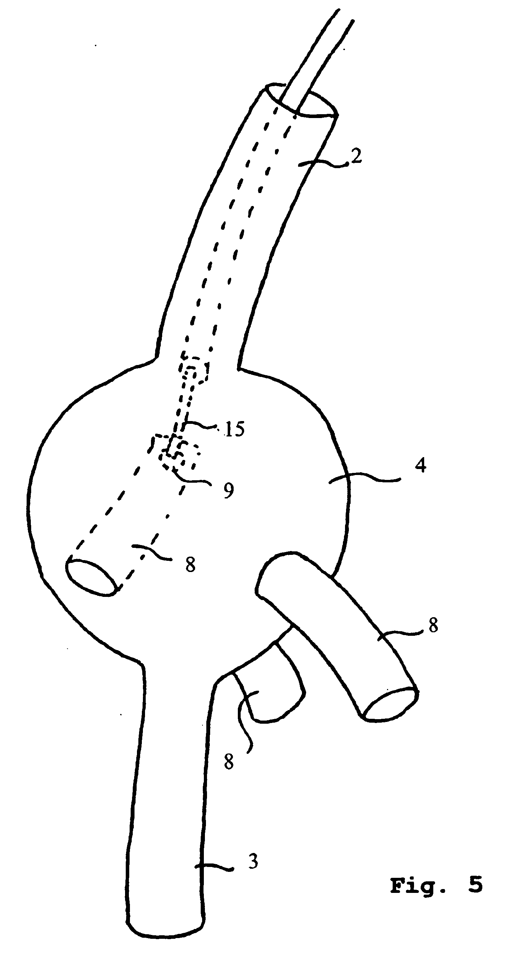

Fig. 5 zeigt das Einstülpen eines schlauchförmigen Fortsatzes 8 mithilfe der endoskopischen Greifzange 15. Diese Prozedur kann unter Verwendung eines Endoskops 14 durchgeführt werden.

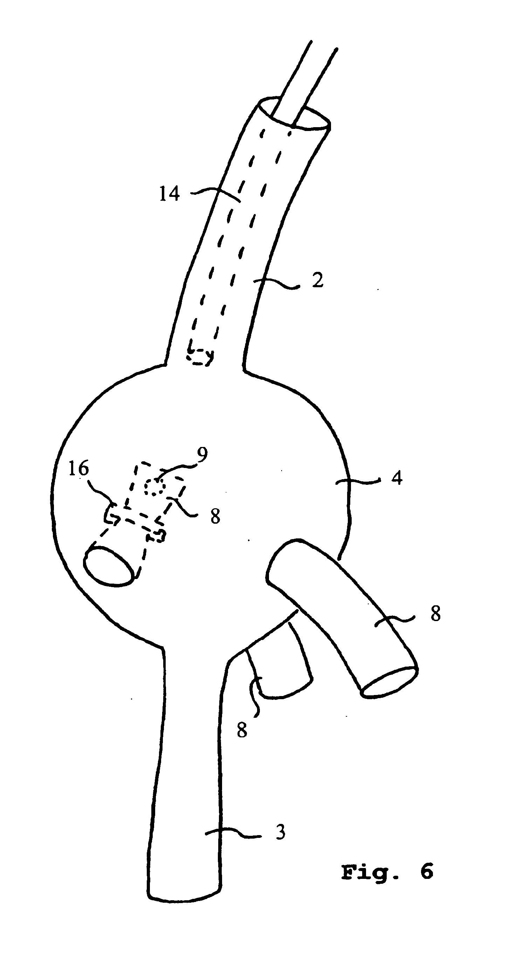

Fig. 6 zeigt den Verschluss eines eingestülpten schlauchartigen Fortsatzes 8. Dazu wird eine mechanische Vorrichtung 16, wie z.B. eine Klammer oder ein Gummiband, an den eingestülpten schlauchartigen Fortsatz appliziert. Diese Prozedur kann unter Verwendung eines Endoskops 14 durchgeführt werden.

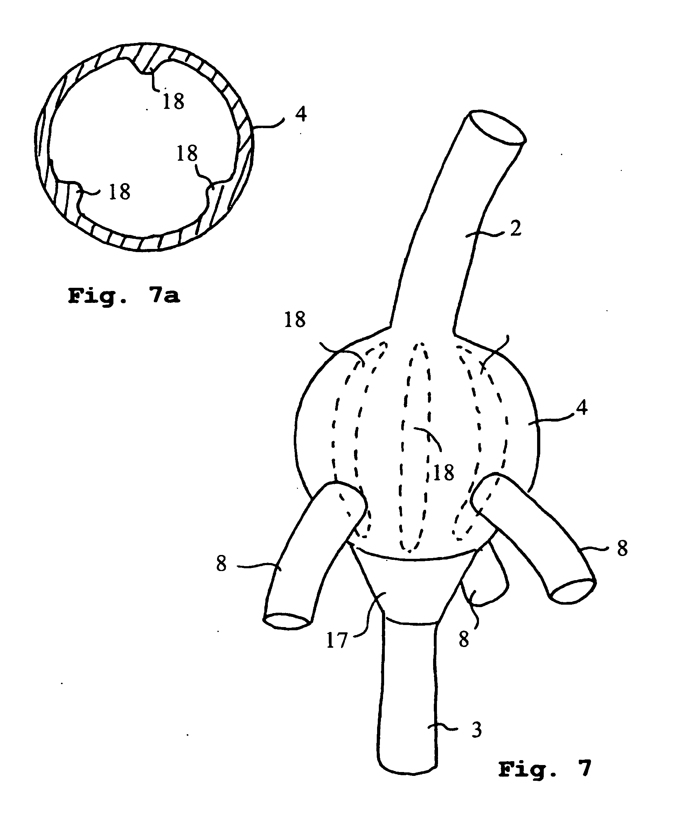

Fig. 7 zeigt das Implantat in einer vorteilhaften Ausführung. Der zweite röhrenartige Endabschnitt 3 weist eine mechanisch feste, trichterförmige Struktur auf. Fig. 7a zeigt den Hohlkörper des Implantats in der Schnittansicht. Die Wand des Hohlkörpers weist eine longitudinale Struktur 18 auf, die das Verbiegen der Wand des Hohlkörpers parallel zur axialen Ebene verhindern soll. Damit soll das Auftreten von quer zur Flussrichtung von Substanzen liegenden Falten in der Wand des Hohlkörpers vermieden werden, um eine störungsfreie Passage durch das Implantat zu ermöglichen. Darüber hinaus werden Substanzen im Hohlkörper vom ersten Endabschnitt in Richtung des zweiten Endabschnitts geführt.

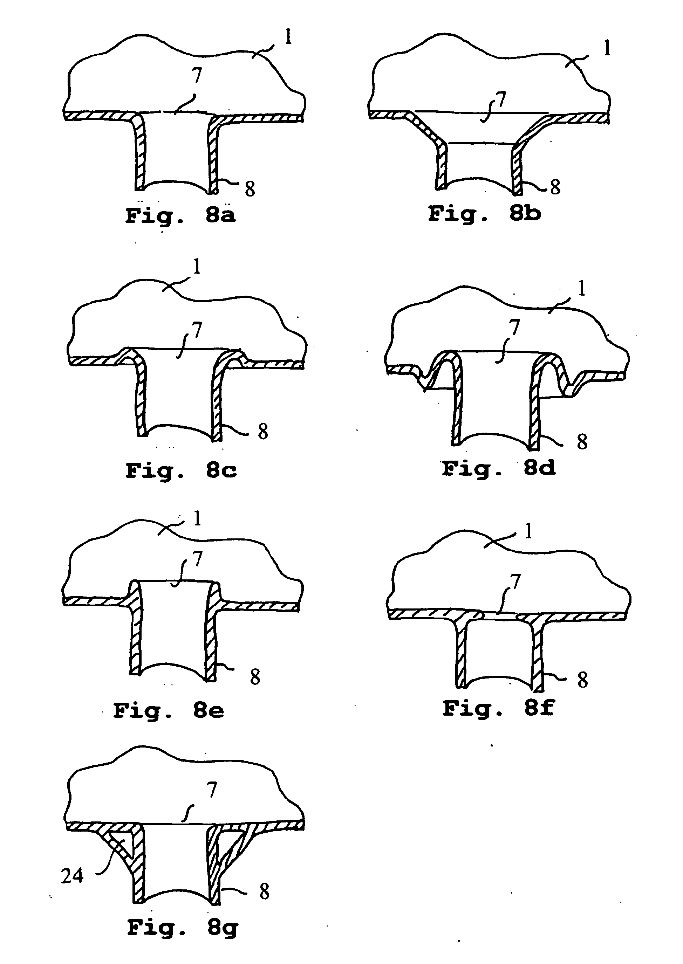

Fig. 8a bis 8f zeigen unterschiedliche Varianten der seitlichen Öffnung 7.

Fig. 8a zeigt eine seitliche Öffnung 7 in einer einfachen Variante.

Fig. 8b zeigt eine seitliche Öffnung 7, die am Übergang zwischen dem schlauchartigen Fortsatz 8 und dem Hohlkörper 1 einen trichterförmigen Endabschnitt 19 aufweist.

Fig. 8c zeigt eine seitliche Öffnung 7, die am Übergang zwischen dem schlauchartigen Fortsatz 8 und Hohlkörper 1 eine Falte 20 aufweist.

Fig. 8d zeigt eine seitliche Öffnung 7, die am Übergang zwischen dem schlauchartigen Fortsatz 8 und dem Hohlkörper 1 eine Doppelfalte 21 aufweist.

Fig. 8e zeigt eine seitliche Öffnung 7, die am Übergang zwischen dem schlauchartigen Fortsatz 8 und dem Hohlkörper 1 einen Wulst 22 parallel zu dem schlauchartigen Fortsatz aufweist.

Fig. 8f zeigt eine seitliche Öffnung 7, die am Übergang zwischen dem schlauchartigen Fortsatz 8 und dem Hohlkörper 1 einen Wulst 23 senkrecht zu dem schlauchartigen Fortsatz aufweist.

Fig. 8g zeigt eine seitliche Öffnung 7, die am Übergang zwischen dem schlauchartigen Fortsatz 8 und dem Hohlkörper 1 einen Hohlraum 24 aufweist.

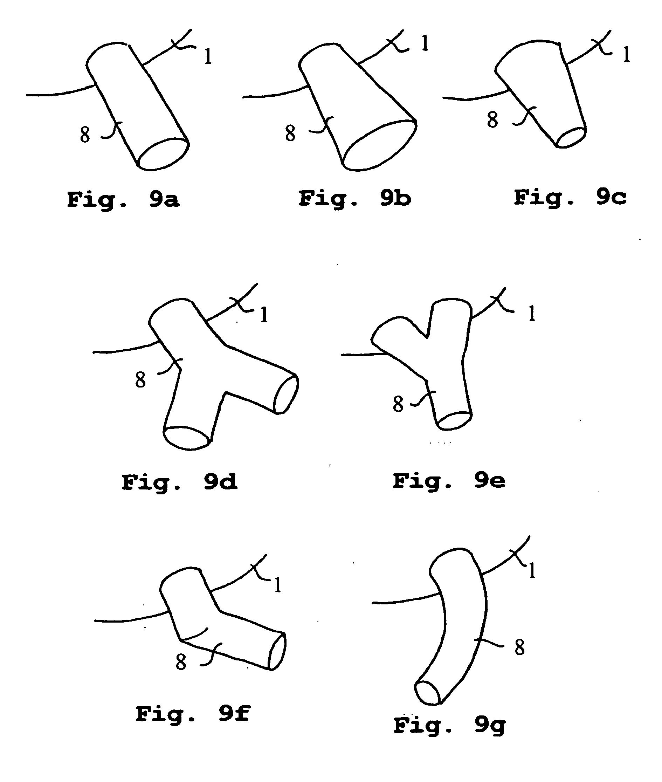

Fig. 9a bis 9g zeigen unterschiedliche Varianten des schlauchartigen Fortsatzes.

Fig. 9a zeigt einen schlauchartigen Fortsatz 8, der über seine Länge einen konstanten Querschnitt aufweist.

Fig. 9b und 9c zeigen einen schlauchartigen Fortsatz 8, der eine trichterartige Form aufweist.

Fig. 9d und 9e zeigen einen schlauchartigen Fortsatz 8, der eine Verzweigung aufweist.

Fig. 9f zeigt einen schlauchartigen Fortsatz 8, der einen Knick aufweist.

Fig. 9g zeigt einen schlauchartigen Fortsatz 8, der eine Biegung aufweist.

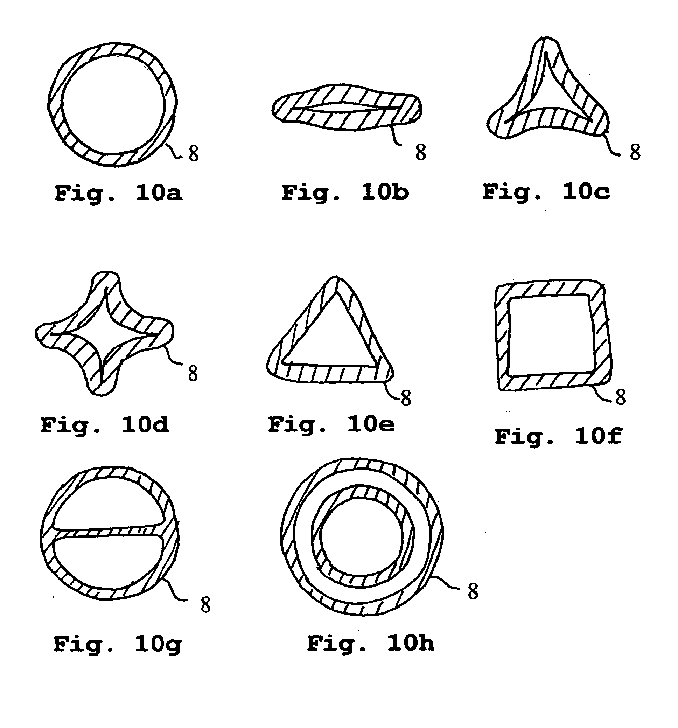

Fig. 10a bis 10h zeigen unterschiedliche Varianten des Querschnitts des schlauchartigen Fortsatzes 8.

Fig. 10a zeigt einen kreisrunden Querschnitt des schlauchartigen Fortsatzes 8.

Fig. 10b zeigt einen schlitzartigen Querschnitt des schlauchartigen Fortsatzes 8.

Fig. 10c und 10d zeigen Varianten eines sternförmigen Querschnitts des schlauchartigen Fortsatzes 8.

Fig. 10e und 10f zeigen Varianten eines eckigen Querschnitts des schlauchartigen Fortsatzes 8.

Fig. 10g und 10h zeigen Varianten eines segmentierten Querschnitts des schlauchartigen Fortsatzes 8.

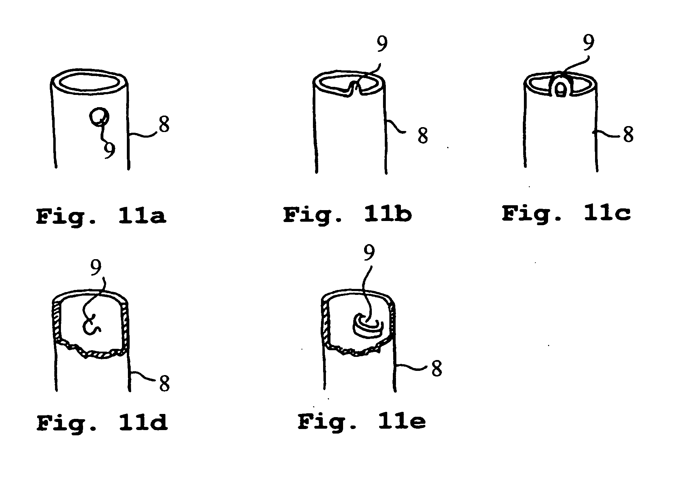

Fig. 11a bis 11e zeigen unterschiedliche Varianten einer Struktur, mit deren Hilfe das Greifen des schlauchartigen Fortsatzes mit einem mechanischen Instrument erleichtert wird.

Fig. 11a zeigt eine Variante der Struktur 9, die eine Öffnung in dem schlauchartigen Fortsatz 8 aufweist.

Fig. 11b zeigt eine Variante der Struktur 9, die einen Wulst an der Stirnseite des schlauchartigen Fortsatzes aufweist.

Fig. 11c zeigt eine Variante der Struktur 9, die eine Lasche an der Stirnseite des schlauchartigen Fortsatzes aufweist.

Fig. 11d zeigt eine Variante der Struktur 9, die einen Wulst auf der Innenseite des schlauchartigen Fortsatzes aufweist.

Fig. 11e zeigt eine Variante der Struktur 9, die eine Lasche auf der Innenseite des schlauchartigen Fortsatzes aufweist.

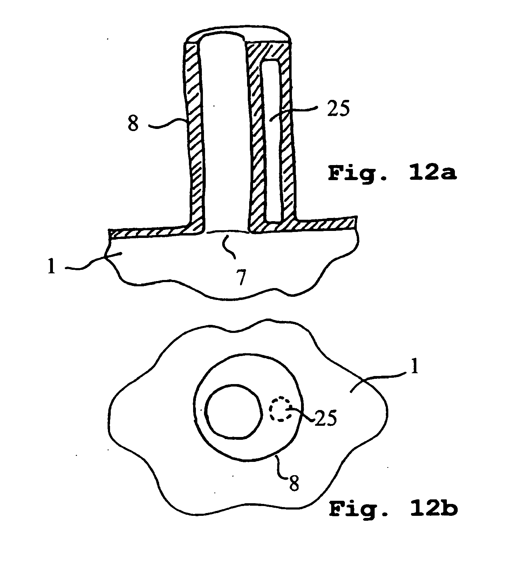

Fig. 12a und Fig. 12b zeigen den longitudinalen Hohlraum 25 in der Außenwand des schlauchartigen Fortsatzes 8 in der Seitenansicht (Fig. 12a) und der Draufsicht (Fig. 12b). Durch Befüllen des longitudinalen Hohlraumes mit einem Medium oder durch Einbringen eines Fremdkörpers kann die Steifigkeit des schlauchartigen Fortsatzes 8 beeinflusst werden, wodurch wiederum die Passage von Substanzen durch die entsprechende seitliche Öffnung 7 variiert werden kann.

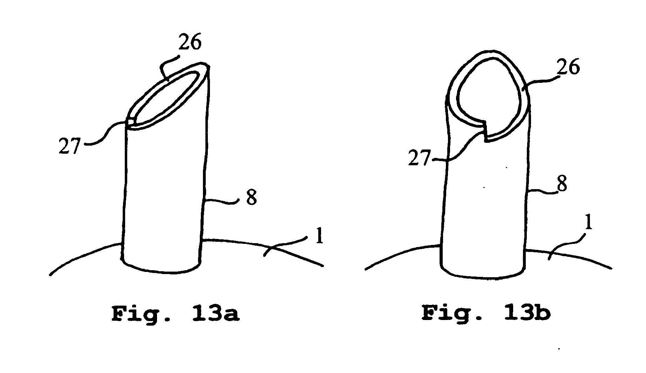

Fig. 13a und 13b zeigen einen schlauchartigen Fortsatz 8, der eine schräge Stirnseite 26 und eine Stufe 27 in der schrägen Stirnseite 26 aufweist in der Seitenansicht (Fig. 13a) und der Frontansicht (Fig. 13b). Durch diese Konstruktion soll eine Vorzugsrichtung erreicht bzw. eine Blockade beim Einstülpen des schlauchartigen Fortsatzes verhindert werden.

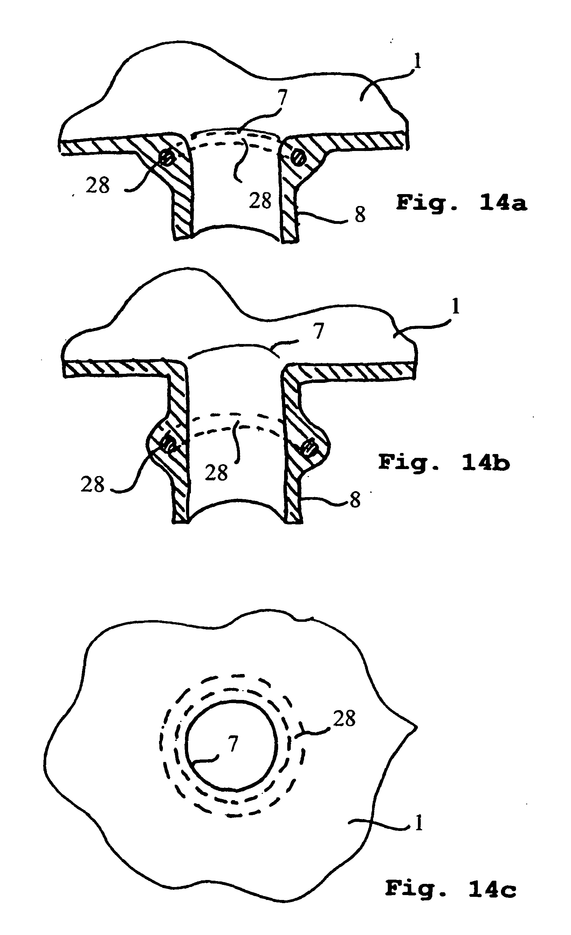

Fig. 14a bis 14c zeigen zwei Variationen eines internen Verschlussmechanismus' für den zumindest teilweisen Verschluss einer seitlichen Öffnung 7 bzw. eines schlauchartigen Fortsatzes 8 in der Seitenansicht im Schnitt (Fig. 14a und 14b) sowie in der Draufsicht (Fig. 14c). Hierzu ist eine Vorrichtung 28, z.B. eine Schlinge, in die Wand des Hohlkörpers 1 oder des schlauchartigen Fortsatzes 8 eingebracht, deren Durchmesser durch Manipulation von außen variiert werden kann.

Das Implantat gemäß der vorliegenden Erfindung hat folgende Vorteile.

Der gesamte Eingriff kann mit minimal invasiven Operationsverfahren durchgeführt werden, wodurch die Belastungen und Risiken für den Patienten minimiert werden. Das Implantat ist im Allgemeinen wieder aus dem Patienten entfernbar, wodurch der Eingriff im Gegensatz zum Gastric-Bypass mit einfachen Mitteln reversibel ist. Später auftretende Komplikationen können daher durch Entfernen des Implantats beseitigt werden.

Ein weiterer Vorteil im Hinblick auf den Gastric-Bypass ist, dass das Abgrenzen einer Magentasche mit Klammernahtvorrichtungen entfällt. Somit entstehen durch das Implantat an dieser Stelle keine Nähte, die später zu Komplikationen führen könnten.

Darüber hinaus ist eine starke Gewichtsreduzierung möglich, da die Nahrung den Magen und den Zwölffingerdarm nur noch teilweise passiert, Verdauungssäfte aus Gallenblase und Pankreas erst später in Kontakt mit der Nahrung treten können und außerdem der obere Dünndarmbereich umgangen wird. Dadurch ist die aktive Darmoberfläche stark reduziert und die Nährstoffaufnahme massiv vermindert. Andererseits ist der letztgenannte therapeutische Effekt im Gegensatz zum Gastric-Bypass durch post-operative Einstellung am Implantat skalierbar. Dadurch können unerwünschte Nebenwirkungen, die z.B. durch übermäßige Malabsorption entstehen, reduziert werden.

Zusätzlich bleibt im Gegensatz zu bisherigen Gastric-Bypass-Verfahren ein Zugang zum Magen erhalten, so dass dieser Bereich für gastroskopische Untersuchungen weiterhin zur Verfügung steht.

## Patentansprüche

1. Medizinisches Implantat bestehend aus einem in den Magen eines Patienten implantierbaren Hohlkörper (1) mit einem ersten röhrenartigen Endabschnitt (2) und einem zweiten röhrenartigen Endabschnitt (3), wobei der erste röhrenartige Endabschnitt (2) so bemessen ist, dass er in die Speiseröhre des Patienten einpassbar ist, und der zweite röhrenartige Endabschnitt (3) so bemessen ist, dass er mit einer Dünndarmschlinge des Patienten dichtend verbindbar ist, **dadurch gekennzeichnet, dass** der Hohlkörper (1) zwischen dem ersten röhrenartigen Endabschnitt (2) und dem zweiten röhrenartigen Endabschnitt (3) mindestens eine dritte seitliche Öffnung (7) aufweist, die derart gestaltet ist, dass sie mindestens einen schlauchartigen Fortsatz (8) aufweist.

2. Medizinisches Implantat gemäß Anspruch 1, **dadurch gekennzeichnet, dass** der erste und zweite röhrenartige Endabschnitt (2, 3) Fixiervorrichtungen (13) zum Fixieren der Endabschnitte an Organteilen des Patienten aufweist.

3. Medizinisches Implantat gemäß Anspruch 1, **dadurch gekennzeichnet, dass** der Hohlkörper (1) zwischen dem ersten und dem zweiten röhrenartigen Endabschnitt (2, 3) ein hohles Mittelteil (4) aufweist, das relativ zu den Endabschnitten (2, 3) ausgebaucht ist und mit diesen in Verbindung ist.

4. Medizinisches Implantat gemäß Anspruch 3, **dadurch gekennzeichnet, dass** das hohle Mittelteil (4) aus einem flexiblen Material oder einer flexiblen Struktur besteht.

5. Medizinisches Implantat gemäß Anspruch 3, **dadurch gekennzeichnet, dass** mindestens eine seitliche Öffnung (3) an dem hohlen Mittelteil (4) gemäß Anspruch 3 lokalisiert ist.

6. Medizinisches Implantat gemäß Anspruch 1, **dadurch gekennzeichnet, dass** der schlauchförmige Fortsatz (8) der seitlichen Öffnung (7) aus einem leicht verformbaren Material oder einer leicht verformbaren Struktur besteht.

7. Medizinisches Implantat gemäß Anspruch 1, **dadurch gekennzeichnet, dass** der schlauchartige Fortsatz (8) trichterförmig gestaltet ist.

8. Medizinisches Implantat gemäß Anspruch 1, **dadurch gekennzeichnet, dass** der schlauchartige Fortsatz (8) eine Verzweigung aufweist.

9. Medizinisches Implantat gemäß Anspruch 1, **dadurch gekennzeichnet, dass** der schlauchartige Fortsatz (8) mindestens einen Knick aufweist.

10. Medizinisches Implantat gemäß Anspruch 1, **dadurch gekennzeichnet, dass** der schlauchartige Fortsatz (8) mindestens eine Biegung aufweist.

11. Medizinisches Implantat gemäß Anspruch 1, **dadurch gekennzeichnet, dass** der schlauchartige Fortsatz (8) einen kreisrunden Querschnitt aufweist.

12. Medizinisches Implantat gemäß Anspruch 1, **dadurch gekennzeichnet, dass** der schlauchartige Fortsatz (8) einen schlitzartigen Querschnitt aufweist.

13. Medizinisches Implantat gemäß Anspruch 1, **dadurch gekennzeichnet, dass** der schlauchartige Fortsatz (8) einen sternförmigen Querschnitt aufweist.

14. Medizinisches Implantat gemäß Anspruch 1, **dadurch gekennzeichnet, dass** der schlauchartige Fortsatz (8) einen eckigen Querschnitt aufweist.

15. Medizinisches Implantat gemäß Anspruch 1, **dadurch gekennzeichnet, dass** der schlauchartige Fortsatz (8) einen segmentierten Querschnitt aufweist.

16. medizinisches Implantat gemäß Anspruch 1, **dadurch gekennzeichnet, dass** der schlauchartige Fortsatz (8) der seitlichen Öffnung (7) mindestens eine Struktur aufweist, die das Greifen mit einem mechanischen Instrument erleichtert.

17. Medizinisches Implantat gemäß Anspruch 16, **dadurch gekennzeichnet, dass** der schlauchartige Fortsatz (8) der seitlichen Öffnung (7) mindestens eine Struktur aufweist, die das Greifen mit einem mechanischen Instrument erleichtert, wobei diese Struktur eine Öffnung (9) in der Außenwand des schlauchartigen Fortsatzes (8) ist.

18. Medizinisches Implantat gemäß Anspruch 16, **dadurch gekennzeichnet, dass** der schlauchartige Fortsatz (8) der seitlichen Öffnung (7) mindestens eine Struktur aufweist, die das Greifen mit einem mechanischen Instrument erleichtert, wobei diese Struktur eine Wulst auf der Stirnseite des schlauchartigen Fortsatzes (8) ist.

19. Medizinisches Implantat gemäß Anspruch 16, **dadurch gekennzeichnet, dass** der schlauchartige Fortsatz (8) der seitlichen Öffnung (7) mindestens eine Struktur aufweist, die das Greifen mit einem mechanischen Instrument erleichtert, wobei diese Struktur eine Lasche auf der Stirnseite des schlauchartigen Fortsatzes (8) ist.

20. Medizinisches Implantat gemäß Anspruch 16, **dadurch gekennzeichnet, dass** der schlauchartige Fortsatz (8) der seitlichen Öffnung (7) mindestens eine Struktur aufweist, die das Greifen mit einem mechanischen Instrument erleichtert, wobei diese Struktur eine Wulst auf der Innenseite des schlauchartigen Fortsatzes (8) ist.

21. Medizinisches Implantat gemäß Anspruch 16, **dadurch gekennzeichnet, dass** der schlauchartige Fortsatz (8) der seitlichen Öffnung (7) mindestens eine Struktur aufweist, die das Greifen mit einem mechanischen Instrument erleichtert, wobei diese Struktur eine Lasche auf der Innenseite des schlauchartigen Fortsatzes (8) ist.

22. Medizinisches Implantat gemäß Anspruch 16, **dadurch gekennzeichnet, dass** die seitliche Öffnung (7) trichterförmig gestaltet ist.

23. Medizinisches Implantat gemäß Anspruch 16, **dadurch gekennzeichnet, dass** die seitliche Öffnung (7) eine Wulst aufweist.

24. Medizinisches Implantat gemäß Anspruch 16, **dadurch gekennzeichnet, dass** die seitliche Öffnung (7) mindestens eine Faltung aufweist.

25. Medizinisches Implantat gemäß Anspruch 16, **dadurch gekennzeichnet, dass** die seitliche Öffnung (7) einen Hohlraum aufweist, in den ein Medium einbringbar ist.

26. Medizinisches Implantat gemäß Anspruch 25, **dadurch gekennzeichnet, dass** durch Einbringen des Mediums in den Hohlraum der Durchmesser der seitlichen Öffnung (7) veränderbar ist.

27. Medizinisches Implantat gemäß Anspruch 1, **dadurch gekennzeichnet, dass** die Außenrand des schlauchartigen Fortsatzes (8) einen Hohlraum aufweist.

28. Medizinisches Implantat gemäß Anspruch 27, **dadurch gekennzeichnet, dass** in den Hohlraum in der Außenwand des schlauchartigen Fortsatzes (8) ein Medium einbringbar ist.

29. Medizinisches Implantat gemäß Anspruch 27, **dadurch gekennzeichnet, dass** in den Hohlraum in der Außenwand des schlauchartigen Fortsatzes (8) ein Fremdkörper einbringbar ist.

30. Medizinisches Implantat gemäß Anspruch 27 sowie mindestens einem der Ansprüche 28 und 29, **dadurch gekennzeichnet, dass** durch Einbringen eines Mediums oder eines Fremdkörpers in den Hohlraum in der Außenwand des schlauchartigen Fortsatzes (8) die Steifigkeit des schlauchartigen Fortsatzes (8) variierbar ist.

31. Medizinisches Implantat gemäß Anspruch 1, **dadurch gekennzeichnet, dass** eine Stirnseite des schlauchartigen Fortsatzes (8) in einer Ebene liegt, die nicht senkrecht zur Achse des schlauchartigen Fortsatzes (8) liegt.

32. Medizinisches Implantat gemäß Anspruch 31, **dadurch gekennzeichnet, dass** eine Stirnseite des schlauchartigen Fortsatzes (8) eine Stufe aufweist.

33. Medizinisches Implantat gemäß Anspruch 1, **dadurch gekennzeichnet, dass** der schlauchartige Fortsatz (8) der seitlichen öffnung (7) zumindest teilweise verschließbar ist.

34. Medizinisches Implantat gemäß Anspruch 33, **dadurch gekennzeichnet, dass** der schlauchartige Fortsatz (8) eine Vorrichtung (16) aufweist, die zumindest einen lokalen öffnungsdurchmesser des schlauchartigen Fortsatzes (8) einstellt.

35. Medizinisches Implantat gemäß den Anspruch 34, **dadurch gekennzeichnet, dass** die Vorrichtung (16) von Außen einstellbar ist.

36. Medizinisches Implantat gemäß Anspruch 1, **dadurch gekennzeichnet, dass** der zweite röhrenartige Endabschnitt (3) eine mechanisch feste, trichterförmig Struktur aufweist.

37. Medizinisches Implantat gemäß Anspruch 1, **dadurch gekennzeichnet, dass** die Wand des Hohlkörpers eine Struktur aufweist, die dem Verbiegen parallel zur axialen Ebene einen höheren mechanischen Widerstand entgegensetzen als dem Verbiegen senkrecht zur axialen Ebene.

## Claims

1. Medical implant comprising a hollow member (1) which can be implanted in the stomach of a patient and which has a first tubular end portion (2) and a second tubular end portion (3), the first tubular end portion (2) being sized in such a manner that it can be fitted into the oesophagus of the patient, and the second tubular end portion (3) being sized in such a manner that it can be connected to a small intestine loop of the patient in a sealing manner, **characterised in that** the hollow member (1) between the first tubular end portion (2) and the second tubular end portion (3) has at least a third lateral opening (7) which is constructed in such a manner that it has at least one hose-like continuation (8).

2. Medical implant according to claim 1, **characterised in that** the first and the second tubular end portions (2, 3) have fixing devices (13) for fixing the end portions to organ parts of the patient.

3. Medical implant according to claim 1, **characterised in that** the hollow member (1) between the first and the second tubular end portions (2, 3) has a hollow central portion (4) which protrudes relative to the end portions (2, 3) and is connected thereto.

4. Medical implant according to claim 3, **characterised in that** the hollow central portion (4) comprises a flexible material or a flexible structure.

5. Medical implant according to claim 3, **characterised in that** at least one lateral opening (7) is located on the hollow central portion (4) according to claim 3.

6. Medical implant according to claim 1, **characterised in that** the hose-like continuation (8) of the lateral opening (7) comprises a readily deformable material or a readily deformable structure.

7. Medical implant according to claim 1, **characterised in that** the hose-like continuation (8) is constructed in a funnel-like manner.

8. Medical implant according to claim 1, **characterised in that** the hose-like continuation (8) has a branch.

9. Medical implant according to claim 1, **characterised in that** the hose-like continuation (8) has at least one kink.

10. Medical implant according to claim 1, **characterised in that** the hose-like continuation (8) has at least one bend.

11. Medical implant according to claim 1, **characterised in that** the hose-like continuation (8) has a circular cross-section.

12. Medical implant according to claim 1, **characterised in that** the hose-like continuation (8) has a slot-like cross-section.

13. Medical implant according to claim 1, **characterised in that** the hose-like continuation (8) has a star-like cross-section.

14. Medical implant according to claim 1, **characterised in that** the hose-like continuation (8) has an angular cross-section.

15. Medical implant according to claim 1, **characterised in that** the hose-like continuation (8) has a segmented cross-section.

16. Medical implant according to claim 1, **characterised in that** the hose-like continuation (8) of the lateral opening (7) has at least one structure which facilitates gripping with a mechanical instrument.

17. Medical implant according to claim 16, **characterised in that** the hose-like continuation (8) of the lateral opening (7) has at least one structure which facilitates gripping with a mechanical instrument, this structure being an opening (9) in the outer wall of the hose-like continuation (8).

18. Medical implant according to claim 16, **characterised in that** the hose-like continuation (8) of the lateral opening (7) has at least one structure which facilitates gripping with a mechanical instrument, this structure being a protuberance on the front end of the hose-like continuation (8).

19. Medical implant according to claim 16, **characterised in that** the hose-like continuation (8) of the lateral opening (7) has at least one structure which facilitates gripping with a mechanical instrument, this structure being a flap on the front end of the hose-like continuation (8).

20. Medical implant according to claim 16, **characterised in that** the hose-like continuation (8) of the lateral opening (7) has at least one structure which facilitates gripping with a mechanical instrument, this structure being a protuberance on the inner side of the hose-like continuation (8).

21. Medical implant according to claim 16, **characterised in that** the hose-like continuation (8) of the lateral opening (7) has at least one structure which facilitates gripping with a mechanical instrument, this structure being a flap on the inner side of the hose-like continuation (8).

22. Medical implant according to claim 16, **characterised in that** the lateral opening (7) is constructed in a funnel-like manner.

23. Medical implant according to claim 16, **characterised in that** the lateral opening (7) has a protuberance.

24. Medical implant according to claim 16, **characterised in that** the lateral opening (7) has at least one fold.

25. Medical implant according to claim 16, **characterised in that** the lateral opening (7) has a hollow cavity into which a medium can be introduced.

26. Medical implant according to claim 25, **characterised in that**, by introducing the medium into the hollow cavity, the diameter of the lateral opening (7) can be adjusted.

27. Medical implant according to claim 1, **characterised in that** the outer wall of the hose-like continuation (8) has a hollow cavity.

28. Medical implant according to claim 27, **characterised in that** a medium can be introduced into the hollow cavity in the outer wall of the hose-like continuation (8).

29. Medical implant according to claim 27, **characterised in that** a foreign body can be introduced into the hollow cavity in the outer wall of the hose-like continuation (8).

30. Medical implant according to claim 27 and at least either claim 28 or claim 29, **characterised in that**, by introducing a medium or a foreign body into the hollow cavity in the outer wall of the hose-like continuation (8), the rigidity of the hose-like continuation (8) can be varied.

31. Medical implant according to claim 1, **characterised in that** a front end of the hose-like continuation (8) is located in a plane which is not perpendicular relative to the axis of the hose-like continuation (8).

32. Medical implant according to claim 31, **characterised in that** a front end of the hose-like continuation (8) has a step.

33. Medical implant according to claim 1, **characterised in that** the hose-like continuation (8) of the lateral opening (7) can be at least partially closed.

34. Medical implant according to claim 33, **characterised in that** the hose-like continuation (8) has a device (16) which adjusts at least a local opening diameter of the hose-like continuation (8).

35. Medical implant according to claim 34, **characterised in that** the device (16) can be adjusted from the outer side.

36. Medical implant according to claim 1, **characterised in that** the second tubular end portion (3) has a mechanically strong, funnel-like structure.

37. Medical implant according to claim 1, **characterised in that** the wall of the hollow member has a structure which provides a higher level of mechanical resistance against bending parallel with the axial plane than against bending perpendicularly relative to the axial plane.

## Revendications

1. Implant médical, composé d'un corps creux (1), susceptible d'être implanté dans l'estomac d'un patient, avec un premier tronçon d'extrémité (2) de type tubulaire et un deuxième tronçon d'extrémité (3) de type tubulaire, le premier tronçon d'extrémité (2) de type tubulaire étant de dimensions telles qu'il est susceptible d'être ajusté dans l'oesophage du patient, et le deuxième tronçon d'extrémité (3) de type tubulaire étant de dimensions telles qu'il est susceptible d'être relié de manière étanche à l'anse grêle du patient, **caractérisé en ce que** le corps creux (1) présente, entre le premier tronçon d'extrémité (2) de type tubulaire et le deuxième tronçon d'extrémité (3) de type tubulaire, au moins une troisième ouverture latérale (7), configurée de manière qu'elle présente au moins un prolongement (8) du genre d'un tuyau.

2. Implant médical selon la revendication 1, **caractérisé en ce que** le premier et le deuxième tronçon d'extrémité (2, 3) de type tubulaire présentent des dispositifs de fixation (13), pour fixer les tronçons d'extrémité à des parties d'organes du patient.

3. Implant médical selon la revendication 1, **caractérisé en ce que** le corps creux (1) présente, entre le premier et le deuxième tronçon d'extrémité (2, 3) de type tubulaire, une partie médiane (4) creuse, renflée par rapport aux tronçons d'extrémité (2, 3) et reliée à celles-ci.

4. Implant médical selon la revendication 3, **caractérisé en ce que** la partie médiane (4) creuse est composée d'un matériau flexible ou d'une structure flexible.

5. Implant médical selon la revendication 3, **caractérisé en ce qu'**au moins une ouverture latérale (7) est localisée sur la partie médiane (4) creuse selon la revendication 3.

6. Implant médical selon la revendication 1, **caractérisé en ce que** le prolongement (8) en forme de tuyau de l'ouverture latérale (7) est composé d'un matériau aisément déformable ou d'une structure aisément déformable.

7. Implant médical selon la revendication 1, **caractérisé en ce que** le prolongement (8) du genre d'un tuyau est conformé en entonnoir.

8. Implant médical selon la revendication 1, **caractérisé en ce que** le prolongement (8) du genre d'un tuyau présente une ramification.

9. Implant médical selon la revendication 1, **caractérisé en ce que** le prolongement (8) du genre d'un tuyau présente au moins un point d'inflexion.

10. Implant médical selon la revendication 1, **caractérisé en ce que** le prolongement (8) du genre d'un tuyau présente au moins un coudage.

11. Implant médical selon la revendication 1, **caractérisé en ce que** le prolongement (8) du genre d'un tuyau présente une section transversale circulaire.

12. Implant médical selon la revendication 1, **caractérisé en ce que** le prolongement (8) du genre d'un tuyau présente une section transversale d'une genre d'une fente.

13. Implant médical selon la revendication 1, **caractérisé en ce que** le prolongement (8) du genre d'un tuyau présente une section transversale en forme d'étoile.

14. Implant médical selon la revendication 1, **caractérisé en ce que** le prolongement (8) du genre d'un tuyau présente une section transversale polygonale.

15. Implant médical selon la revendication 1, **caractérisé en ce que** le prolongement (8) du genre d'un tuyau présente une section transversale segmentée.

16. Implant médical selon la revendication 1, **caractérisé en ce que** le prolongement (8), du genre d'un tuyau, de l'ouverture (7) latérale présente au moins une structure facilitant la saisie avec un instrument mécanique.

17. Implant médical selon la revendication 16, **caractérisé en ce que** le prolongement (8), du genre d'un tuyau, de l'ouverture (7) latérale présente au moins une structure facilitant la saisie avec un instrument mécanique, cette structure étant une ouverture (4) ménagée dans la paroi extérieure du prolongement (8) du genre d'un tuyau.

18. Implant médical selon la revendication 16, **caractérisé en ce que** le prolongement (8), du genre d'un tuyau, de l'ouverture (7) latérale présente au moins une structure facilitant la saisie avec un instrument mécanique, cette structure étant un bourrelet réalisé sur la face frontale du prolongement (8) du genre d'un tuyau.

19. Implant médical selon la revendication 16, **caractérisé en ce que** le prolongement (8), du genre d'un tuyau, de l'ouverture (7) latérale présente au moins une structure facilitant la saisie avec un instrument mécanique, cette structure étant une patte réalisée sur la face frontale du prolongement (8) du genre d'un tuyau.

20. Implant médical selon la revendication 16, **caractérisé en ce que** le prolongement (8), du genre d'un tuyau, de l'ouverture (7) latérale présente au moins une structure facilitant la saisie avec un instrument mécanique, cette structure étant un bourrelet réalisé sur la face intérieure du prolongement (8) du genre d'un tuyau.

21. Implant médical selon la revendication 16, **caractérisé en ce que** le prolongement (8), du genre d'un tuyau, de l'ouverture (7) latérale présente au moins une structure facilitant la saisie avec un instrument mécanique, cette structure étant une patte réalisé sur la face intérieure du prolongement (8) du genre d'un tuyau.

22. Implant médical selon la revendication 16, **caractérisé en ce que** l'ouverture (7) latérale est conformée en entonnoir.

23. Implant médical selon la revendication 16, **caractérisé en ce que** l'ouverture (7) latérale présente un bourrelet.

24. Implant médical selon la revendication 16, **caractérisé en ce que** l'ouverture (7) latérale présente au moins un plissement.

25. Implant médical selon la revendication 16, **caractérisé en ce que** l'ouverture (7) latérale présente un espace creux, dans lequel un fluide peut être introduit.

26. Implant médical selon la revendication 25, **caractérisé en ce que** le diamètre de l'ouverture (7) latérale est modifiable par introduction du fluide dans l'espace creux.

27. Implant médical selon la revendication 1, **caractérisé en ce que** la paroi extérieure du prolongement (8) du genre d'un tuyau présente un espace creux.

28. Implant médical selon la revendication 27, **caractérisé en ce qu'**un fluide est susceptible d'être introduit dans l'espace creux, dans la paroi extérieure du prolongement (8) du genre d'un tuyau.

29. Implant médical selon la revendication 27, **caractérisé en ce qu'**un corps étranger est susceptible d'être introduit dans l'espace creux, dans la paroi extérieure du prolongement (8) du genre d'un tuyau.

30. Implant médical selon la revendication 27, ainsi qu'au moins l'une des revendications 28 et 29, **caractérisé en ce que** la rigidité du prolongement (8) du genre d'un tuyau est modifiable par introduction d'un fluide ou d'un corps étranger dans l'espace creux, dans la paroi extérieure du prolongement (8) du genre d'un tuyau.

31. Implant médical selon la revendication 1, **caractérisé en ce qu'**une face frontale du prolongement (8) du genre d'un tuyau est située dans un plan non perpendiculaire à l'axe du prolongement (8) du genre d'un tuyau.

32. Implant médical selon la revendication 31, **caractérisé en ce qu'**une face frontale du prolongement (8) du genre d'un tuyau présente un étagement.

33. Implant médical selon la revendication 1, **caractérisé en ce que** le prolongement (8), du genre d'un tuyau, de l'ouverture latérale (7) est obturable au moins partiellement.

34. Implant médical selon la revendication 33, **caractérisé en ce que** le prolongement (8) du genre d'un tuyau présente un dispositif (16) réglant au moins un diamètre d'ouverture local du prolongement (8) du genre d'un tuyau.

35. Implant médical selon la revendication 34, **caractérisé en ce que** le dispositif (16) est réglable de l'extérieur.

36. Implant médical selon la revendication 1, **caractérisé en ce que** le deuxième tronçon d'extrémité (3) de type tubulaire présente une structure mécaniquement rigide, en forme d'entonnoir.

37. Implant médical selon la revendication 1, **caractérisé en ce que** la paroi du corps creux présente une structure opposant à la déformation parallèlement au plan axial une résistance mécanique plus élevée qu'à la déformation perpendiculairement au plan axial.
